# EUROPEAN PATENT APPLICATION

(11) **EP 2 490 027 A1**
(43) Date of publication of application: **22.08.2012**
(21) Application number: 11154532.3
(22) Date of filing: 15.02.2011
(51) Int. Cl.: G01N 33/68

(54) **Means and methods for diagnosing pregnancy complications based on GDF-15 and PlGF/sFlt1**

(71) Applicant: Roche Diagnostics GmbH, 68305 Mannheim (DE); F. Hoffmann-La Roche AG, 4070 Basel (CH)
(72) Inventor: Hess, Georg, 55130 Mainz (DE); Horsch, Andrea, 68259 Mannheim (DE); Zdunek, Dietmar, 82327 Tutzing (DE)
(74) Representative: Dick, Alexander

(57) **Abstract**

The present invention relates to the field of diagnostic measures. Specifically, it relates to a method for diagnosing whether a pregnant subject suffering from preeclampsia or having a predisposition therefor is at risk of experiencing a premature termination of pregnancy, or not, said method comprising the steps of determining the amount of PlGF and GDF-15 and, preferably, sFlt1 in a sample of the said subject and comparing the determined amounts to reference amounts for PlGF and GDF-15 and, preferably, sFlt1, whereby an increased risk of experiencing premature termination of pregnancy is to be diagnosed. Further encompassed is a diagnostic device and a kit for carrying out the aforementioned method.

## Description

The present invention relates to the field of diagnostic measures. Specifically, it relates to a method for diagnosing whether a pregnant subject suffering from preeclampsia or having a predisposition therefor is at risk of experiencing a premature termination of pregnancy, or not, said method comprising the steps of determining the amount of PlGF and GDF-15 and, preferably, sFlt1 in a sample of the said subject and comparing the determined amounts to reference amounts for PlGF and GDF-15 and, preferably, sFlt1, whereby an increased risk of experiencing premature termination of pregnancy is to be diagnosed. Further encompassed is a diagnostic device and a kit for carrying out the aforementioned method.

Pregnancy may be complicated in different ways, it is on one hand associated with pregnancy related mortality of the pregnant woman and also associated with increased morbidity and mortality of the newborn. Maternal mortaliy at a rate of 14,5 per 100.000 live births, is more frequent in pregnant women above the age of 39 years and may be caused by hemorrage, thrombotic pulmonary embolism, infections, cardiomyopathy and cardiovascular and noncardiovascular conditions as well as hypertensive disorders among which pre-eclampsia is the most frequent.(Berg 2010, Obstretics and Gynecology: 116: 1302 - 1309). In newborns prematurity is associated with increased mortality and morbidity. Preterm infants with very low birth weight (VLBW) (below 1500 g) and extremely low birth weight (ELBW) (below 1000 g) do have the highest risk of morbidity and mortality. Weight relative to duration of gestation may add as a component of risk, however weight on its own is no proxy for fetal maturity or immaturity (McCormick 2011, Annu Rev Public Health 32: 15.1 - 15.3). Infant mortality increases sharply with decreasing gestational age from 175 per 1000 (below 32 weeks of gestation) to 2.4/1000 at 37 - 41 weeks of gestation (Mc Cormick, loc cit.). Key components of neonatal risk include intracranial bleeding, white matter damage and sensory impairment, lung immaturity and respiratory distress syndrome as well as sepsis. (McCormick et al). It is also well appreciated that morbidity of prematurity of infants extends into later age and is associated with growth retardation, impaired pulmonary function, learning disability, abnormal behavior, including inattentive and hyperactive behaviour leading as a result to impaired quality of life (Mc Cormick, loc cit.).

One disease affecting both maternal mortality and being involved in permaturity of the newborn is preeclampsia.

The most important complication in pregnancy is preeclampsia. Pre-eclampsia complicates approximately 2 - 8 percent of all pregnancies and is- as indicated - a major contributor to maternal mortality worldwide and is also associated with prematurity and intrauterine growth restriction (Duley 2009, Semin Perinatol: 33: 130 - 37). Preeclampsia is generally defined as new hypertension /diastolic blood pressure of equal or more than 90 mmHg at or after 20 weeks gestation. Definition of preeclampsia is subject to debate and differs significantly among societies (Steegers 2010, Lancet 376: 631 - 644).

The pathogenesis of preeclampsia is largely unknown. It is believed to be caused by disturbed placenta function associated with impaired remodelling of the spiral artery (which can be visualized by ultrasound). Flow defects occurring in the process of the development of preeclampsia are associated with ischemia which ultimately results in the release of anti-angiogenic factors into the circulations such as sFlT1 and endoglin (Karumachi S.A. et al in Lindheimer M.D., Roberts J.M., Cunningham F.G. Eds, Chelsley's hypertensive disorders in pregnancy. Amsterdam Academic Press, Elesevier 2009: 87 - 103).

Treatment of choice of preeclampsia is termination of pregnancy either by vaginal or caesarean delivery. As discussed above maternal risks and fetal viability are significantly impaired in case of preeclampsia before gestational week 30, thus attempts should be made to delay delivery and to improve survival of the newborn, Survival of the newborn can be improved by the application of betamethasone which accelerates fetal lung maturation and reduces the risk of respiratory distress syndrome (Steegers, loc cit.).

Decision making as to termination of pregnancy or short term delivery appears to be complex, Includes clinical symptoms and severity of preeclampsia (which is difficult to determine ( Steegers, loc cit.) and assumed maturity of the unborn. As discussed there are no tests that would indicate maturity of the unborn (see above).

Placenta growth factor (PlGF) and its receptor (sFlT1) to increase until week 38 - 41 of pregnancy and then fall with the initiation of labor. In normal, uneventful pregnancy sFlT1 increases when PlGF decreases and, thus, is an indicator of imminent delivery. In preeclampsia this event occurs prematurely and as early as week 20 of pregnancy.(Karumanchi, loc cit.).

Growth differentiation factor 15 (GDF 15) as a member of the TGF beta familiy has been reported to be an indicator of successful establishment and maintenance of pregnancy (Marjono 2003, Placenta: 24: 100 - 108). Recently, however, it has been suggested that GDF 15 levels might reflect cardiovascular risk as has been found in nonpregnant subjects (Sugulle 2009, Hypertension 54: 106 - 112; Brown 2002, Lancet 359: 2159 - 2163).

Thus, there is an urgent need to refine the diagnosis of normal uneventful pregnancy and its possible complications preferentially preeclampsia.

The technical problem underlying the present invention can be seen as the provision of means and methods for reliably and efficiently diagnosing in a subj ect suffering from preeclampsia or having a predisposition therefor whether said subject is at risk of experiencing a premature termination of pregnancy. The technical problem is solved by the embodiments characterized in the claims and herein below.

The present invention, thus, relates to a method for diagnosing whether a pregnant subject suffering from preeclampsia or having a predisposition therefor is at risk of experiencing a premature termination of pregnancy, or not, said method comprising the steps of:
a) determining the amount of PlGF and GDF-15 in a sample of the said subject; and
b) comparing the amounts determined in step a) to reference amounts for PlGF and GDF-15, whereby an increased risk of experiencing a premature termination of pregnancy is to be diagnosed.

Preferably, it is diagnosed whether a pregnant subject suffering from preeclampsia or having a predisposition therefor is at risk of experiencing a premature termination of pregnancy, or not, by carrying out the further step of c) diagnosing whether a pregnant subject suffering from preeclampsia or having a predisposition therefor is at risk of experiencing a premature termination of pregnancy, or not, based on the result of the comparison carried out in step b).

The method of the present invention, preferably, is an ex vivo method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or diagnosis based on said comparison in step (b). More preferably, the method is carried out entirely in an automated manner. In such a case, the diagnostic result which is established in step b) is generated in a suitable output format so that it can be used as an aid for establishing the final clinical diagnosis by, e.g., a medical practitioner.

The term "preeclampsia" as used herein refers to a medical condition which is characterized by hypertension and proteinuria. Preeclampsia occurs in pregnant females and the hypertension is also referred to as pregnancy-induced hypertension. Preferably, the pregnancy-induced hypertension is identified to be present in a subject by two blood pressure measurements of 140 mmHg (systolic) to 90 mmHg (diastolic) or more, wherein said two measurements have been made at least 6 hours apart. Proteinuria is, preferably, identified to be present by 300 mg/dL protein or more in a 24-hour urine sample. Pre-eclampsia may progress to eclampsia, a life-threatening disorder characterized by the appearance of tonic-clonic seizures or coma. Preeclampsia is often asymptomatic. However, symptoms associated with preeclampsia are epigastric pain, which reflects hepatic involvement and is typical of the HELLP syndrome, and edema or swelling, in particular of the hands and face. Further details of preeclampsia and the accompanying symptoms are to be found in Steegers, loc. cit.. Preeclampsia occurs in up to 10% of pregnancies usually in the second or third trimester. However, some females develop preeclampsia as early as in week 20 of gestation. A subject having a predisposition for preeclampsia is a subject suffering from overweight, adiposity, hypertension, autoimmune diseases such as Lupus erythematosus, thrombophilias or diabetes mellitus. Furthermore, elderly females who are pregnant for the first time do also exhibit a predisposition for developing preeclampsia. The likelihood for developing preeclampsia, however, is decreasing with the number of pregnancies.

The term "premature termination of pregnancy" as used herein refers to any termination of pregnancy which occurs prior to the physiological birth of a mature child. Thus, the term encompasses abortion of a death embryo or fetus or premature birth (also sometimes referred to as premature delivery) of a viable fetus. Abortion as used herein refers to a termination of the pregnancy by removal or expulsion of the death embryo or fetus. Preferably, the term as used herein refers to abortion induced by the preeclampsia and occurs spontaneously. A spontaneous abortion is also referred to as miscarriage. Similarly, premature birth refers to a termination of pregnancy by expulsion of a viable fetus. The premature birth, also preferably, occurs spontaneously and is preeclampsia induced. A viable fetus is, preferably, a fetus which developed until the 20^{th} week of gestation or later. Therefore, it is preferably envisaged that a premature birth occurs as a premature termination of pregnancy for a pregnant subject from the 20^{th} week of gestation onwards, while for a pregnant subject being prior to the 20^{th} week of gestation, abortion occurs.

Accordingly, by diagnosing in accordance with the method of the present invention whether a pregnant subject is at risk of experiencing a premature termination of pregnancy, a method is provided which serves for a pregnant subject being prior to the 20^{th} week of gestation for diagnosing whether the said subject is at risk of experiencing abortion, or not. Moreover, a method is provided which serves for a pregnant subject being in the 20^{th} week of gestation or later for diagnosing whether the said subject is at risk of experiencing premature birth, or not.

The term "at risk" as used in the method of the present invention refers to an increased likelihood for experiencing a premature termination of pregnancy within a certain prognostic time window. Preferably, the said prognostic time window in accordance with the present invention is the time window starting at the time when the test sample has been obtained until the calculated or real day of the physiological birth of the mature child. A subject will be at risk of experiencing a premature termination of pregnancy if the likelihood of experiencing such a premature termination of pregnancy for the investigated subject is increased in a statistically significant manner compared to the prevalence (i.e. the normal likelihood) of premature termination of pregnancy for a pregnant subject suffering from preeclampsia or having a predisposition therefor. The normal likelihood of premature termination of pregnancy can be, preferably, determined in a cohort of subjects suffering from preeclampsia or a predisposition therefor as the mean or average likelihood for a premature termination of pregnancy. Whether, or not, the likelihood differs therefrom in a statistically significant manner, can be determined by standard statistical measures including those referred to elsewhere herein.

The term "subject" as used herein relates to animals, preferably mammals, and, more preferably, humans. The subject according to the present invention shall suffer from preeclampsia or shall have a predisposition therefor as described elsewhere herein. The subject referred to herein is a pregnant subject and, thus, preferably, a female subject. Moreover, preferably, the subject is not suffering from an acute systemic infection, such as sepsis or SIRS, diabetes and/or a cardiovascular disease or disorder other than preeclampsia. More preferably, the subject is except for the preeclampsia apparently healthy.

The term "diagnosing" as used herein means assessing whether a subject as referred to in accordance with the method of the present invention is at risk of experiencing a premature termination of pregnancy, or nor. Diagnosing whether a subject is at risk of experiencing premature termination of pregnancy, or not, shall also include establishing a prognosis of whether a subject as referred to herein will experience a premature termination of pregnancy, or not. As will be understood by those skilled in the art, such an assessment is usually not intended to be correct for 100% of the subjects to be diagnosed. The term, however, requires that the assessment is correct for a statistically significant portion of the subjects (e.g. a cohort in a cohort study). Thus, the method of the present invention, however, at least provides an aid for establishing a final clinical diagnosis. Whether a portion is statistically significant, can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma, urine or serum and, most preferably, blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein. More preferably, the sample is derived from a subject being in any one of week of gestation 20 to 38.

The term "Growth and Differentiation Factor-15" or "GDF-15" relates to a polypeptide being a member of the transforming growth factor beta (TGFβ) cytokine superfamily. The terms polypeptide, peptide and protein are used interchangeable throughout this specification. GDF-15 was originally cloned as macrophage-inhibitory cytokine-1 and later also identified as placental transforming growth factor-β, placental bone morphogenetic protein, non-steroidal anti-inflammatory drug-activated genre-1, and prostate-derived factor (Hromas 1997, Biochim Biophys Acta 1354:40-44; Lawton 1997, Gene 203:17-26; Yokoyama-Kobayashi 1997, J Biochem (Tokyo), 122:622-626; Paralkar 1998, J Biol Chem 273:13760-13767). Similar to other TGF-β-related cytokines, GDF-15 is synthesized as an inactive precursor protein, which undergoes disulfide-linked homodimerization. Upon proteolytic cleavage of the N-terminal pro-peptide, GDF-15 is secreted as a ~28 kDa dimeric protein (Bauskin 2000, Embo J 19:2212-2220). Amino acid sequences for GDF-15 are disclosed in WO99/06445, WO00/70051, WO2005/113585 Bottner 1999, Gene 237: 105-111, Baek 2001, Mol Pharmacol 59: 901-908, Hromas loc cit, Paralkar loc cit, Morrish 1996, Placenta 17:431-441 or Yokoyama-Kobayashi loc cit.. GDF-15 as used herein encompasses also variants of the aforementioned specific GDF-15 polypeptides. Such variants have at least the same essential biological and immunological properties as the specific GDF-15 polypeptides. In particular, they share the same essential biological and immunological properties if they are detectable by the same specific assays referred to in this specification, e.g., by ELISA assays using polyclonal or monoclonal antibodies specifically recognizing the said GDF-15 polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific GDF-15 polypeptides. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific GDF-15 polypeptides or the aforementioned types of variants as long as these fragments have the essential immunological and biological properties as referred to above. Such fragments may be, e.g., degradation products of the GDF-15 polypeptides. Further included are variants which differ due to posttranslational modifications such as phosphorylation or myristylation.

The term "PlGF (Placental Growth Factor)" as used herein refers to a placenta derived growth factor which is a polypeptide having 149 amino acids in length and being highly homologous (53% identity) to the platelet-derived growth factor-like region of human vascular endothelial growth factor (VEGF). Like VEGF, PlGF has angiogenic activity in vitro and in vivo. For example, biochemical and functional characterization of PlGF derived from transfected COS-1 cells revealed that it is a glycosylated dimeric secreted protein which is able to stimulate endothelial cell growth in vitro (Maqlione1993, Oncogene 8(4):925-31). Preferably, PlGF refers to human PlGF, more preferably, to human PlGF having an amino acid sequence as shown in Genebank accession number P49763, GI: 17380553 (Genebank is available from the NCBI, USA under www.ncbi.nlm.nih.gov/entrez).Furthermore, the term encompasses variants of said specific human PlGF. Such variants have at least the same essential biological and immunological properties as the specific PlGF polypeptide. Variants are deemed to share the same essential biological and immunological properties if they are detectable by the same specific assays referred to in this specification, e.g., by ELISA assays using polyclonal or monoclonal antibodies specifically recognizing the said PlGF polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific PlGF polypeptides. The degree of identity between two amino acid sequences can be determined by algorithms well known in the art and described elsewhere herein. Variants referred to above may be allelic variants or any other species specific homologs, paralogs, or orthologs. Moreover, the variants referred to herein include fragments of the specific PLGF polypeptides or the aforementioned types of variants as long as these fragments have the essential immunological and biological properties as referred to above. Such fragments may be, e.g., degradation products or splice variants of the PLGF polypeptides. Further included are variants which differ due to posttranslational modifications such as phosphorylation or myristylation.

Preferably, the amount of sFlt1 is determined in addition to PlGF and GDF-15 in the method of the present invention, wherein said amount of sFlt1 is compared to a reference amount of sFlt1 for diagnosing an increased risk of experiencing premature termination of pregnancy.

The term "soluble Flt1" or "sFlt1" as used herein refers to polypeptide which is a soluble form of the VEGF receptor Flt1. It was identified in conditioned culture medium of human umbilical vein endothelial cells. The endogenous soluble Flt1 (sFlt1) receptor is chromatographically and immunologically similar to recombinant human sFlt1 and binds [125I] VEGF with a comparable high affinity. Human sFlt1 is shown to form a VEGF-stabilized complex with the extracellular domain of KDR/Flk-1 in vitro. Preferably, sFlt1 refers to human sFlt1 as describe in Kendall 1996, Biochem Biophs Res Commun 226(2): 324-328 (for amino acid sequences, see, e.g., also P17948, GI: 125361 for human and BAA24499.1, GI: 2809071 for mouse sFlt1). The term also encompasses variants of the aforementioned human sFlt1 polypeptides. Such variants have at least the same essential biological and immunological properties as the aforementioned sFlt1 polypeptide. In particular, they share the same essential biological and immunological properties if they are detectable by the same specific assays referred to in this specification, e.g., by ELISA assays using polyclonal or monoclonal antibodies specifically recognizing the said sFlt1 polypeptides. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific sFlt-1 polypeptide, preferably over the entire length of the human sFlt1, respectively. The degree of identity between two amino acid sequences can be determined by algorithms well known in the art and described elsewhere herein. Variants referred to above may be allelic variants or any other species specific homologs, paralogs, or orthologs. Moreover, the variants referred to herein include fragments or subunits of the specific sFlt1 polypeptides or the aforementioned types of variants as long as these fragments have the essential immunological and biological properties as referred to above. Such fragments may be, e.g., degradation products of the sFlt-1 polypeptides. Further included are variants which differ due to posttranslational modifications such as phosphorylation or myristylation.

Determining the amount of a peptide or polypeptide referred to in this specification relates to measuring the amount or concentration, preferably, semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay and methods which may utilize labelled molecules in various sandwich, competition, or other assay formats. Such assays are, preferably, based on detection agents such as antibodies which specifically recognize the peptide or polypeptide to be determined. The detection agents shall be either directly or indirectly capable of generating a signal indicating the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Further suitable techniques for the determination of a polypeptide or peptide are described in the following.

First, binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labelled with a detectable label prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labelling may be done by direct or indirect methods. Direct labelling involves coupling of the label directly (covalently or non-covalently) to the ligand. Indirect labelling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labelled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provides the desired assessment in a suitable output format, i.e. the diagnostic result. The said diagnostic result may, preferably, serve as an aid for establishing the final clinical diagnosis by, e.g., a medical practitioner.

Based on the comparison of the amount determined in step a) and the reference amount, it shall be possible to assess whether a subject is at risk of premature termination of pregnancy, or not. Therefore, the reference amount is to be chosen so that either a difference or an identity in the compared amounts allows identifying those test subjects which belong into the group of subjects which are either at risk of premature termination of pregnancy, or not. The method allows either excluding (rule-out) or identifying (rule-in) a subject as a subject being at risk for premature termination of pregnancy, or not. Differences in the amounts, i.e. increases or decreases, as used herein, preferably, are differences which are statistically significant. Whether a difference is statistically significant can be determined by the statistical techniques referred to elsewhere herein. Similarly, an identity in the amounts encompasses identical amounts and those differences in the amounts which are not statistically significant and which are within the standard deviations for a measured parameter.

The term "reference amount" as used herein refers to an amount which allows for allocation of a subject into either (i) the group of subjects being at risk for premature termination of pregnancy or (ii) the group of subjects being not at risk of premature termination of pregnancy. The reference amount applicable for an individual subject may vary depending on various physiological parameters such as age, gender, or subpopulation, as well as on the means used for the determination of the polypeptide or peptide referred to herein. A suitable reference amount may be determined from a reference sample to be analyzed together, i.e. simultaneously or subsequently, with the test sample.

Reference amounts can, in principle, be calculated for a cohort of subjects as specified above based on the average or mean values for a given biomarker by applying standard methods of statistics. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity versus specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention, i.e. a threshold which allows to discriminate between subjects being at increased risk for mortality or those which have a normal risk among a cohort of subjects suffering from acute inflammation can be generated, preferably, by establishing a ROC for said cohort as described above and deriving a threshold amount therefrom. Dependent on a desired sensitivity and specificity for a diagnostic method, the ROC plot allows deriving suitable thresholds. It will be understood that an optimal sensitivity is desired for excluding a subject for being at increased risk (i.e. a rule out) whereas an optimal specificity is envisaged for a subject to be assessed as being at an increased risk (i.e. a rule in).

Preferably, said reference amounts are derived from a pregnant subject or a group thereof known not to suffer from preeclampsia or having a predisposition therefor and, more preferably, from an apparently healthy subject or group thereof. Preferably, said reference amounts are derived from a pregnant subject or group thereof being at the same stage (e.g. trimester, month or week) of gestation as the subject to be tested.

More preferably, indicative for a pregnant subject suffering from preeclampsia or having a predisposition therefor being not at risk of experiencing a premature termination of pregnancy is:
(i) an amount of PlGF which is identical or increased compared to the reference amount for PlGF in combination with an amount of GDF-15 which is identical or increased compared to the reference amount for GDF-15, or
(ii) an amount of PlGF which is identical or decreased compared to the reference amount for PlGF in combination with an amount of GDF-15 which is identical or increased compared to the reference amount for GDF-15 and an amount of sFlt1 which is identical or deceased compared to the reference amount for sFlt1.

More preferably, indicative for a pregnant subject suffering from preeclampsia or having a predisposition therefor is at risk of experiencing a premature termination of pregnancy is:
(i) an amount of PlGF which is decreased compared to the reference amount for PlGF in combination with an amount of GDF-15 which is decreased compared to the reference amount for GDF-15, or
(ii) an amount of PlGF which is decreased compared to the reference amount for PlGF in combination with an amount of GDF-15 which is identical or increased compared to the reference amount for GDF-15 and an amount of sFlt1 which is increased compared to the reference amount for sFlt1.

Preferably, the risk of experiencing an premature termination of pregnancy is higher for the combination of sFlt1 and GDF-15 referred to in (i) than those combinations referred to in (ii).

More preferably, reference amounts which are derived from a pregnant subject or a group thereof known not to suffer from preeclampsia or having a predisposition therefor are the following threshold amounts which reflect the median values for the biomarkers found in pregnant subjects which are not suffering from preeclampsia are (in the order median, 25^{th} percentile - 75^{th} percentile):
Week 18-22 of gestation: PlGF, about 159 (121-494) pg/ml; GDF-15, about 22,600 (19,200-27,300) pg/ml; sFlt1, about 1,482 (1,165-2.073) pg/ml;
Week 23-27 of gestation: PlGF, about 282 (173-494) pg/ml; GDF-15, about 29,800 (25,600-40,500) pg/ml; sFlt1, about 1,702 (1,150-2,228) pg/ml;
Week 28-32 of gestation: PlGF, about 398 pg/ml (285-712); GDF-15, about 50,900 (39,100-61,500) pg/ml; sFlt1, about 1,896 (1,305-2,536) pg/ml;
Week 33-37 of gestation: PlGF, about 301 pg/ml (915-424); GDF-15, about 77,000 (61,250-96,680) pg/ml; sFlt1, about 2,584 (2,109-4,967) pg/ml.

Dependent on the envisage specificity and/or sensitivity, the 25^{th} or 75^{th} percentile values for the respective stages could also be used instead of the median values (see accompanying Examples, below). In particular, the percentile values of the 75^{th} percentiles are envisaged for the method of the present invention.

The term "about" as used herein means +/- 20%, +/- 10%, +/- 5%, +- 2 % or +-/ 1% from the specific values referred to.

In principle, the present invention also relates to a method for diagnosing in a pregnant subject suffering from preeclampsia or having a predisposition therefor whether said subject is at risk or experiencing a premature termination of pregnancy, or not, said method comprising diagnosing whether said subject is at risk or experiencing a premature termination of pregnancy, or not based on a comparison of the amount of the PlGF in combination with GDF-15 and, preferably, sFlt1 determined in a sample of said subject to reference amounts.

Advantageously, it has been found in the studies underlying the present invention that the amount a combination of the biomarkers PlGF, GDF-15 and sFlt1 allow for risk assessment of pregnancy in pregnant subjects suffering from preeclampsia or having a predisposition therefor. This risk assessment of a pregnancy is important for the further therapeutic steps envisaged. For example, if a pregnant subject as referred to herein is at risk of experiencing a premature termination of pregnancy, therapeutic measures will be required such as closer monitoring of the pregnant subject as well as therapeutic measure such as drug administration, preferably, aspirin, oxytocin, prostaglandin E2 or misoprostol administration, or invasive measures, preferably, cesarean or amniotomy. Moreover, dependent on whether a premature abortion or a premature birth is to be expected, therapeutic measures for the developing fetus may be required as well. In particular, if a premature birth is to be expected, the fetus after birth will need special intensive care, such as fetal incubation. Thanks to the present invention, it is possible to more reliably diagnose whether a pregnant subject is at risk of premature termination of pregnancy. Moreover, time consuming, expensive and cumbersome diagnostic measures can be avoided when applying the biomarker-based method of the invention as an aid for diagnosis.

It is to be understood that the definitions and explanations of the terms made above and below apply accordingly for all embodiments described in this specification and the accompanying claims.

In a preferred embodiment of the method of the present invention, said method further comprises the step of recommending a therapeutic measure if an increased risk has been diagnosed.

The term "recommending" as used herein means establishing a proposal for a therapy which could be applied to the subject. However, it is to be understood that applying the actual therapy whatsoever is not comprised by the term. The therapy to be recommended depends on the outcome of the diagnosis provided by the method of the present invention. The recommendation step referred to above can also, preferably, be automated. Preferably, the diagnosis or aid for diagnosis obtained from the step b) of the method of the present invention, i.e. the diagnostic result of the method, will be used to search a database comprising recommendations of therapeutic measures for the individual possible diagnostic results.

Preferably, said therapeutic measure is selected from the group consisting of: drug administration, preferably, aspirin, oxytocin, prostaglandin E2 or misoprostol administration, or invasive measures, preferably, cesarean or amniotomy. More preferably, the aforementioned therapeutic measures are recommended for the pregnant subject if the subject has been diagnosed to be at risk for experiencing premature termination of pregnancy and, more preferably, a premature birth. It will be understood that the aforementioned recommendations shall, preferably, apply for a subject from the 20^{th} week of gestation onwards.

Also preferably and/or in addition, the therapeutic measure may be therapeutic measures for the fetus, and, in particular, fetus incubation and/or drug administration for lung maturation. Preferably, the dug to be administered for lung maturation is betamethasone. The aforementioned therapeutic measures for the fetus are recommended for the developing fetus if the subject has been diagnosed to be at risk for experiencing premature termination of pregnancy. It will be understood that the aforementioned recommendations shall, preferably, apply for a subject from the 20^{th} week after gestation onwards. In particular, it will be understood that, preferably, a fetus which developed at least until week 20 of gestation can be grown to a mature child by fetal incubation. Between the 20^{th} week of gestation and the 34^{th} week of gestation, administration of a drug for lung maturation is, preferably, recommended.

In another preferred embodiment of the method of the present invention, TGF-ß1 is measured instead of PlGF and/or GDF-15.

It follows from the above that the present invention also contemplates the use of a combination of PlGF, GDF-15 and, preferably, sFlt1 or a combination of detection agents therefor for diagnosing whether a pregnant subject suffering from preeclampsia or having a predisposition therefor is at risk of experiencing an premature termination of pregnancy, or not, in a sample of the subject. Also contemplated is the use of detection agents for PlGF, GDF-15 and preferably also sFlt1 for the manufacture of a diagnostic composition or device for whether a pregnant subject suffering from preeclampsia or having a predisposition therefor is at risk of experiencing an premature termination of pregnancy, or not, in a sample of the subject.

The term "detection agent" as used herein refers to an agent which is capable of specifically recognizing and binding to one of the biomarkers referred to above when present in a sample. Moreover, said agent shall allow for direct or indirect detection of the complex formed by the said agent and the biomarker. Direct detection can be achieved by including into the agent a detectable label. Indirect labelling may be achieved by a further agent which specifically binds to the complex comprising the biomarker and the detection agent wherein the said further agent is than capable of generating a detectable signal. Suitable compounds which can be used as detection agents are well known in the art. Preferably, the detection agent is an antibody or aptamere which specifically binds to the biomarker.

The present invention relates to a device for diagnosing whether a pregnant subject suffering from preeclampsia or having a predisposition therefor is at risk of experiencing an premature termination of pregnancy, or not, said device comprising:
a) an analyzing unit comprising a detection agent for PlGF, GDF-15 and, preferably, sFlt1 which allows for the determination of the amounts of PlGF, GDF-15 and, preferably, sFlt1; and
b) an evaluation unit comprising a data processor having implemented an algorithm for comparing the amounts determined by the analyzing unit with reference amounts stored in a database in order to establish a diagnosis indicating whether a pregnant subject suffering from preeclampsia or having a predisposition therefor is at risk of experiencing an premature termination of pregnancy, or not, wherein said reference amounts are derived from a pregnant subject or a group thereof known not to suffer from preeclampsia or having a predisposition therefor and the algorithm is an algorithm as defined above in connection with the method of the invention.

The term "device" as used herein relates to a system comprising the aforementioned units operatively linked to each other as to allow the diagnosis according to the methods of the invention. Preferred detection agents which can be used for the analyzing unit are disclosed elsewhere herein. The analyzing unit, preferably, comprises said detection agents in immobilized form on a solid support which is to be contacted to the sample comprising the biomarkers the amount of which is to be determined. Moreover, the analyzing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference. Suitable references can be derived from samples of subjects to be used for the generation of reference amounts as described elsewhere herein above. The diagnostic results may be given as output of parametric diagnostic raw data, preferably, as absolute or relative amounts. It is to be understood that these data may need interpretation by the clinician. However, also envisage are expert system devices wherein the output comprises processed diagnostic raw data the interpretation of which does not require a specialized clinician. Preferably, the device of the present invention can be used to carry out the aforementioned method of the present invention in an automated manner.

In a preferred embodiment of the device of the present invention, said evaluation unit further comprises a database with recommendations for therapeutic measures and wherein said data processor further having implemented an algorithm which allows for recommending a therapeutic measure if an increased risk has been diagnosed.

The present invention, finally, encompasses a kit for diagnosing whether a pregnant subject suffering from preeclampsia or having a predisposition therefor is at risk of experiencing an premature termination of pregnancy, or not, said kit comprising at least a detection agent for PlGF and GDF-15 and, preferably, also for sFlt1 and, preferably, standards which reflect the reference amounts as derived from a pregnant subject or a group thereof known not to suffer from preeclampsia or having a predisposition therefor.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as a optical storage medium (e.g., a Compact Disc) or directly on a computer or data processing device. Moreover, the kit may, preferably, comprise standards which reflect the reference amounts as described and referred to elsewhere herein in detail. The detection agent is, preferably, immobilized on a carrier, and, preferably, a test stripe.

All references cited in this specification are herewith incorporated by reference with respect to their entire disclosure content and the disclosure content specifically mentioned in this specification.

### EXAMPLES

### Example 1: Determination of PlGF, GDF-15 and sFlt1 in pregnant women suffering from preeclampsia and healthy controls

GDF-15 and sFlt1 were determined with sandwich immuno-assays using analyzers from Roche/Hitachi, Elecsys or COBAS e-series. Each assay comprises two monoclonal antibodies specific for the respective polypeptide. The first of these antibodies is biotinylated and the second one is labelled with a Tris(2,2'-bipyridyl)ruthenium(II)-complex. In a first incubation step both antibodies are incubated with the sample. A sandwich complex comprising the peptide to be determined and the two different antibodies is formed. In a next incubation step streptavidin-coated beads are added to this complex. The beads bind to the sandwich complexes. The reaction mixture is then aspirated into a measuring cell where the beads are magnetically captured on the surface of an electrode. The application of a voltage then induces a chemiluminescent emission from the ruthenium complex which is measured by a photomultiplier. The emitted amount of light is dependent on the amount of sandwich complexes on the electrode. The measuring range of GDF-15 it was 300 pg/ml to 20,000 pg/ml. For sFlt1, amounts between 10 to 85,000 pg/ml could be measured.

PlGF was tested using an Elecsys immunoassay (see also above) which employs two antibodies that are specific for PlGF. The test can be carried out automatically using different Roche analysers including Elecsys 2010 and cobra e411 and cobra e601. The test has a measuring range of 3 to 10,000 pg/ml.

A total of 31 pregnant women who experienced an uneventful pregnancy were followed throughout their pregnancy in order to obtain reference values for pathological conditions and specifically for preeclampsia. Samples were taken between week 8 - 13, 14 - 17, 18 - 22, 23 - 27, 28 - 32, 33 - 37, 38 - 41 and 1, 6 and 24 weeks post partum. Patients with preeclampsia (n= 9) were included into the study as they presented. Values obtained in preeclamptic women were compared to reference values obtained in healthy pregnant women at the respective gestational weeks (see also Table 2, below). Reference values were obtained in apparently healthy women as shown in the Table 1, below.

**Table 1: Reference amounts for PlGF, GDF-15, and sFlt1 in pregnant apparently healthy women (median values are indicated as well the range of the 25^{th} - 75^{th} percentiles (indicated below the median values))**

| Week of gestation: | GDF 15 (pg/ml) | PlGF (pg/ml) | sFlT1 (pg/ml) |
|---|---|---|---|
| 8-13 | 21500 | 29 | 1828 |
| | 16200 - 28290 | 23 - 37 | 1190 - 2107 |
| 14 - 17 | 19950 | 64 | 1573 |
| | 14900 - 25140 | 51- 91 | 1150 - 1963 |
| 18-22 | 22600 | 159 | 1482 |
| | 19200 - 27300 | 121 - 494 | 1165 - 2073 |
| 23 - 27 | 29800 | 282 | 1702 |
| | 25600-40500 | 173 - 494 | 1150 - 2228 |
| 28 - 32 | 50900 | 398 | 1896 |
| | 39100 - 61500 | 285 - 712 | 1305 - 2536 |
| 33 - 37 | 77000 | 301 | 2584 |
| | 61350 -96680 | 195 - 424 | 2109 - 4967 |
| 38-41 | 84180 | 170 | 5960 |
| | 57500- 107900 | 136 - 226 | 4621 - 7085 |
| 1 week pp | 1520 | 14 | 1191 |
| | 1227-1668 | 11 - 23 | 578 - 2119 |
| 6 weeks pp | 520 | 9 | 74 |
| | 481 - 620 | 7-11 | 72 - 106 |
| 24 weeks pp | 470 | 8 | 73 |
| | 360 - 520 | 7-9 | 57-81 |

Data obtained in normal uneventful pregnancy indicate that PlGF increase steadily until week 33 - 37 and then decreases just before delivery, as PlGF decreases and sFlt1 increases which is also interpreted as preparation for delivery. GDF-15 remains steady until week 23 and thereafter increases and reaches peak levels between weeks 33 and 41 indicating that GDF-15 has a key role in maintaining late pregnancy (a time at which PlGF decreases and sFlt1 increases). At weeks 6 and 24 post partum (pp), GDF-15, PlGF and sFlt1 reach levels known to occur in non-pregnant apparently healthy subjects (GDF-15: 580 pg/ml, PlGF 11,3 pg/ml and sFlt1 71 pg/ml (median levels of 149 apparently healthy blood donors)) indicating that GDF-15, PlGF and sFlt1 levels observed in pregnancy are unrelated to cardiac disorders in which all of these markers have been observed to correlate with pathological conditions.

### Example 2: Biomarkers PlGF, GDF-15 and sFlt1 in individual cases of preeclampsia

Patient no.64 presents at week 33 with sFlt1 of 6,656 pg/ml, PlGF of 321 pg/ml and GDF-15 of 90,900 pg/ml. Clinical diagnosis of PE was mild, while sFlt1 indicated imminent delivery GDF-15 levels were within the upper normal range consistent with well maintained pregnancy, she delivered at week 37 without further complication.

Patient no.66 presented at week 28 with PlGF of 64 pg/ml, sFlt1 of 1,497 pg/ml and GDF-15 of 96,775 pg/ml. Low levels of PlGF and normal levels of sFlt1 were consistent with the diagnosis of preeclampsia, however GDF-15 was elevated indicating that the pregnancy was still well maintained. The patient was treated with steroid for one week followed by caeserial section.

Patient no.70 presented at week 33 with PlGF of 126 pg/ml, sFlt1 of 1,793 pg/ml and GDF-15 of 226,800 pg/ml. Similar to case 66 low levels of PlGF were compensated by high levels of GDF-15 indicating the pregnancy was still well maintained, she had an uneventful delivery at week 34.

Patient no.77 presents at week 33 with PlGF of 110 pg/ml, sFlt1 of 8,973 pg/ml and GDF-15 of 61,069 pg/ml, Low PlGF levels were associated with low normal GDF-15 levels but significantly increased sFlt1 levels indicating immanent delivery, she delivered at week 33 by caeserian section.

Patient no.79 presented at week 22 with PlGF of 41 pg/ml, sFlt1 of 1,670 pg/ml of GDF-15 of 64,400 pg/ml. Again, low PlGF levels were counterbalanced by high GDF-15 levels, sFlt1 levels were still within normal. After a one week steroid treatment period delivery was initiated.

Patient no. 96 presented at week 33 with PlGF of 145 pg/ml, sFlt1 of 6,443 pg/ml of GDF-15 of 115,000 pg/ml, again low PlGF levels were associated with increased GDF-15 levels and sFlt1 indicated imminent delivery. Delivery was performed in week 33 by caeserian section.

Patient no.191 presented at week 23 with PlGF of 28 pg/ml, sFlt1 of 14,442 pg/ml and GDF-15 of 22,391 pg/ml. In this case low PlGF levels were not counterbalanced by increased GDF-15 levels, at the same time very high sFlt1 levels prompted immediate delivery.

Patient no.199 presented at week 28 with PlGF of 129 pg/ml, sFlt1 of 10,120 pg/ml and GDF-15 of 36,500 pg/ml. Low levels of PlGF were associated with low levels of GDF-15 and increased levels of sFlT1 indicating that pregnancy was not well maintained and delivery by ceserian section was initiated.

Patient no. 200 presented at week 33 with PlGF of 243 pg/ml, sFlt1 of 8,300 pg/ml and GDF-15 of 100,583 pg/ml. Elevated levels of sFlT1 indicating immanent delivery were associated with normal PlGF levels and slightly increased GDF-15 levels indicating stable pregnancy but imminent delivery.

**Table 2: Summary of the study**

| | | | Measured amounts pg/ml* | | | Reference amounts (median of normal controls) pg/ml | | |
|---|---|---|---|---|---|---|---|---|
| Case no. | Premature termination of pregnancy | Week of gestation | PlGF | GDF-15 | sFlt1 | PlGF | GDF-15 | sFlt1 |
| 64 | no | 33 | **321** | **90,900** | **6,656** | 301 | 77,000 | 2,584 |
| 66 | yes | 28 | 64 | **96,775** | 1,497 | 398 | 50,900 | 1,896 |
| 70 | yes | 33 | 126 | **226,800** | 1,793 | 301 | 50,900 | 1,896 |
| 77 | yes | 33 | 110 | 42,600 | **8,973** | 301 | 50,900 | 1,896 |
| 79 | yes | 22 | 41 | **64,400** | 1,670 | 159 | 22,600 | 1,482 |
| 96 | yes | 33 | 145 | **115,000** | **6,443** | 301 | 50,900 | 1,896 |
| 191 | yes | 23 | 28 | 22,391 | **14,442** | 282 | 29,800 | 1,702 |
| 199 | yes | 28 | 129 | 36,500 | **10,120** | 398 | 50,900 | 1,896 |
| 200 | yes | 33 | 243 | **100,583** | **8,300** | 301 | 50,900 | 1,896 |

| | | | | | | | | |
|---|---|---|---|---|---|---|---|---|
| *Measured amounts being increased with respect to the reference are indicated bold, decreased amounts in normal | | | | | | | | |

To the extent data were available at weeks 6 and 24 post partum, sFlt1, PlGF and GDF-15 values returned to normal values (as shown above) and thus fail to indicate cardiovascular sequelae of preeclampsia.

The results obtained in preeclampsia compared to the results obtained in normal pregnancy indicate that low PlGF levels are frequently compensated by increased GDF-15 levels indicating that pregnancy is still in a stable state and does not require immediate intervention. This is different when both, PlGF and GDF-15, decrease or PlGF is decreased and GDF-15 is not increased. Moreover, in order to refine the diagnosis and in terms of decision marking sFlt1 levels can become relevant, too. In particular, sFlt1 helps to refine the diagnosis in cases where low PlGF levels are compensated by GDF-15 levels into cases where the pregnancy is still in a stable status and cases with imminent birth or abortion.

The results obtained by the combined determination according to the method of the invention exemplified herein above provide additional information regarding the pregnancy of pregnant women suffering from preeclampsia compared to a sFlt1/PlGF ratio assay.

**Table 3: sFlt1/PlGF ratios in healthy pregnant women**

| Week of gestation | sFlT1/PlGF of median (25^{th} and 75^{th} percentiles) |
|---|---|
| 8 - 13 | 62.8 (35.8 - 86.0) |
| 14 - 17 | 25.4 (15,8 - 30.2) |
| 18 - 22 | 10.4 (7 - 13.7) |
| 23 - 27 | 5.5 (3.2 - 8.9) |
| 28 - 32 | 4.4 (3.0 - 8.0) |
| 33 - 37 | 10.4 (4.9 - 20.1) |
| 38 - delivery | 36.8 (23 - 51.4) |
| 1 Wo pp | 63.1 (37.2 - 89.3) |
| 6 Wo pp | 9.7 (8.7 - 13.3) |
| 24 Wo pp | 10.6 (7.8 - 12.2) |

**Table 4: sFlt1/PlGF ratios for the individual cases and diagnostic result**

| Case | sFlt1/PlGF ratio | Diagnosis of preeclampsia based on ratio | Risk for premature termination of pregnancy determined based on the method of the invention (Table 2) |
|---|---|---|---|
| Case 64 | 20.7 | No | No |
| Case 66 | 23.4 | No | Yes |
| Case 70 | 14.2 | Yes | Yes |
| Case 77 | 81.5 | Yes | Yes |
| Case 79 | 40.7 | Yes | Yes |
| Case 96 | 44.4 | Yes | Yes |
| Case 191 | 515.8 | Yes | Yes |
| Case 199 | 78.4 | Yes | Yes |
| Case 200 | 34.2 | Yes | Yes |

Based on the sFlt1/PlGF ratios, only patients suffering from preeclampsia can be identified. However, the combined determination of the GDF-15 in combination with PlGF and sFlt1 improves the currently existing diagnostic measures since the patients suffering from preeclampsia could also be distinguished into risk and non-risk subgroups with respect to a premature termination of pregnancy associated with said preeclampsia.

## Claims

1. A method for diagnosing whether a pregnant subject suffering from preeclampsia or having a predisposition therefor is at risk of experiencing a premature termination of pregnancy, or not, said method comprising the steps of:
a) determining the amount of PlGF and GDF-15 in a sample of the said subject; and
b) comparing the amounts determined in step a) to reference amounts for PlGF and GDF-15, whereby an increased risk of experiencing premature termination of pregnancy is to be diagnosed.

2. The method of claim 1, wherein said sample is derived from a subject being in any one of week of gestation 20 to 38.

3. The method of claim 1 or 2, wherein the amount of sFlt1 is determined in addition to PlGF and GDF-15 and wherein said amount of sFlt1 is compared to a reference amount of sFlt1 for diagnosing an increased risk of experiencing a premature termination of pregnancy.

4. The method of any one of claims 1 to 3, wherein said reference amounts are derived from a pregnant subject or a group thereof known not to suffer from preeclampsia or not having a predisposition therefor.

5. The method of claim 4, wherein:
(i) an amount of PlGF which is identical or increased compared to the reference amount for PlGF in combination with an amount of GDF-15 which is identical or increased compared to the reference amount for GDF-15, or
(ii) an amount of PlGF which is identical or decreased compared to the reference amount for PlGF in combination with an amount of GDF-15 which is identical or increased compared to the reference amount for GDF-15 and an amount of sFlt1 which is identical or deceased compared to the reference amount for sFlt1 is indicative for a pregnant subject suffering from preeclampsia or having a predisposition therefor being not at risk of experiencing a premature termination of pregnancy.

6. The method of claim 4, wherein:
(i) an amount of PlGF which is decreased compared to the reference amount for PlGF in combination with an amount of GDF-15 which is decreased compared to the reference amount for GDF-15, or
(ii) an amount of PlGF which is decreased compared to the reference amount for PlGF in combination with an amount of GDF-15 which is identical or increased compared to the reference amount for GDF-15 and an amount of sFlt1 which is increased compared to the reference amount for sFlt1 is indicative for a pregnant subject suffering from preeclampsia or having a predisposition therefor is at risk of experiencing a premature termination of pregnancy.

7. The method of claim 6, wherein the risk of experiencing a premature termination of pregnancy is higher for the combination of PlGF and GDF-15 referred to in (i) than those combinations referred to in (ii).

8. The method of any one of claims 1 to 7, wherein said method further comprises the step of recommending a therapeutic measure if an increased risk has been diagnosed.

9. The method of claim 8, wherein said therapeutic measure is selected from the group consisting of: drug administration, preferably, aspirin, oxytocin, prostaglandin E2 or misoprostol administration, or invasive measures, preferably, cesarean or amniotomy.

10. The method of any one of claims 1 to 9, wherein said sample is a blood, plasma, serum or urine sample.

11. Use of a combination of PlGF, GDF-15 and sFlt1 or a combination of detection agents therefor for diagnosing whether a pregnant subject suffering from preeclampsia or having a predisposition therefor is at risk of experiencing a premature termination of pregnancy, or not, in a sample of the subject.

12. A device for diagnosing whether a pregnant subject suffering from preeclampsia or having a predisposition therefor is at risk of experiencing a premature termination of pregnancy, or not, said device comprising:
a) an analyzing unit comprising a detection agent for PlGF, GDF-15 and sFlt1 which allows for the determination of the amounts of PlGF, GDF-15 and sFlt1; and
b) an evaluation unit comprising a data processor having implemented an algorithm for comparing the amounts determined by the analyzing unit with reference amounts stored in a database in order to establish a diagnosis indicating whether a pregnant subject suffering from preeclampsia or having a predisposition therefor is at risk of experiencing an premature termination of pregnancy, or not, wherein said reference amounts are derived from a pregnant subject or a group thereof known not to suffer from preeclampsia or having a predisposition therefor and the algorithm is an algorithm as defined in claim 5 or 6.

13. The device of claim 12, wherein said evaluation unit further comprises a data base with recommendations for therapeutic measures and wherein said data processor further having implemented an algorithm which allows for recommending a therapeutic measure if an increased risk has been diagnosed.

14. A kit for diagnosing whether a pregnant subject suffering from preeclampsia or having a predisposition therefor is at risk of experiencing a premature termination of pregnancy, or not, said kit comprising at least a detection agent for PlGF and GDF-15 and, preferably, also for sFlt1 and, preferably, standards which reflect the reference amounts as derived from a pregnant subject or a group thereof known not to suffer from preeclampsia or having a predisposition therefor.
